# EUROPEAN PATENT APPLICATION

(11) **EP 2 745 845 A1**
(43) Date of publication of application: **25.06.2014**
(21) Application number: 12306622.7
(22) Date of filing: 19.12.2012
(51) Int. Cl.: A61K 39/21, A61P 31/18, C07K 14/705

(54) **A method for preventing or treating an HIV infection**

(71) Applicant: Centre Hospitalier Universitaire de Bordeaux, 33404 Talence Cedex (FR)
(72) Inventor: Fleury, Hervé, 33000 BORDEAUX (FR)
(74) Representative: Gallois, Valérie

(57) **Abstract**

The present invention relates to a method for determining whether an epitope is susceptible to elicit a T-cell response to human immunodeficiency virus (HIV) in a subject affected with HIV infection based on the proviral sequences and HLA alleles of the subject. It also relates to a method for preparing a vaccine and for selecting a patient for a defined vaccine.

SEQUENCE LISTING

<110>  Centre Hospitalier Universitaire de Bordeaux

<120>  A method for preventing or treating an HIV infection

<130>  B1517EP

<160>  57

<170>  PatentIn version 3.5

<210>  1
<211>  18
<212>  DNA
<213>  artificial sequence

<220>
<223>  GAG First PCR 1st amplicon_Primer 5'

<400>  1
gactagcgga ggctagaa

<210>  2
<211>  24
<212>  DNA
<213>  artificial sequence

<220>
<223>  GAG First PCR 1st amplicon_Primer 3'

<400>  2
tttggtcctt gtcttatgtc caga

<210>  3
<211>  29
<212>  DNA
<213>  artificial sequence

<220>
<223>  GAG First PCR 2nd amplicon_Primer 5'

<400>  3

ataatccacc tatcccagta ggagaaatt

<210>  4
<211>  29
<212>  DNA
<213>  artificial sequence

<220>
<223>  GAG First PCR 2nd amplicon_Primer 3'

<400>  4
atgcttttat tttttcttct gtcaatggc

<210> 5
<211> 18
<212> DNA
<213> artificial sequence

<220>
<223> GAG Second PCR 1st amplicon_Primer 5′

<400> 5
gactagcgga ggctagaa

<210> 6
<211> 23
<212> DNA
<213> artificial sequence

<220>
<223> GAG Second PCR 1st amplicon_Primer 3′

<400> 6
gttctaggtg atatggcctg atg

<210> 7
<211> 25
<212> DNA
<213> artificial sequence

<220>
<223> GAG Second PCR 2nd amplicon_Primer 5′

<400> 7
cacctagaac tttaaatgca tgggt

<210> 8
<211> 24
<212> DNA
<213> artificial sequence

<220>
<223> GAG Second PCR 2nd amplicon_Primer 3′

<400> 8
tttggtcctt gtcttatgtc caga

<210> 9
<211> 29
<212> DNA
<213> artificial sequence

<220>
<223> GAG Second PCR 3rd amplicon_Primer 5′

<400> 9
ataatccacc tatcccagta ggagaaatt

<210> 10
<211> 18
<212> DNA
<213> artificial sequence

<220>
<223> GAG Second PCR 3rd amplicon_Primer 3

<400> 10
aggggtcgtt gccaaaga

<210> 11
<211> 28
<212> DNA
<213> artificial sequence

<220>
<223> GAG Second PCR 4th amplicon_Primer 5′

<400> 11
tcagagcaga ccagagccaa cagcccca

<210> 12
<211> 30
<212> DNA
<213> artificial sequence

<220>
<223> GAG Second PCR 4th amplicon_Primer 3′

<400> 12
aatgctttta ttttttcttc tgtcaatggc

<210> 13
<211> 27
<212> DNA
<213> artificial sequence

<220>
<223> NEF First PCR_Primer 5′

<400> 13
gccacagcca tagcagtagc tgagggg

<210> 14
<211> 29
<212> DNA
<213> artificial sequence

<220>
<223> NEF First PCR_Primer 3′

<400> 14
ccagtacagg caaaaagcag ctgcttata

<210>  15
<211>  29
<212>  DNA
<213>  artificial sequence

<220>
<223>  NEF Second PCR_Primer 5'

<400>  15
cctagaagaa taagacaggg cttggaaag

<210>  16
<211>  26
<212>  DNA
<213>  artificial sequence

<220>
<223>  NEF Second PCR_Primer 3'

<400>  16
acgcctccct ggaaagtccc cagcgg

<210>  17
<211>  20
<212>  DNA
<213>  artificial sequence

<220>
<223>  POL First PCR_Primer 5'

<400>  17
agtaggacct acacctgtca

<210>  18
<211>  22
<212>  DNA
<213>  artificial sequence

<220>
<223>  POL First PCR_Primer 3'

<400>  18
ctgttagtgc tttggttcct ct

<210>  19
<211>  35
<212>  DNA
<213>  artificial sequence

<220>
<223>  POL Second PCR 1st amplicon_Primer 5'

<400>  19
ttggttgcac tttaaatttt cccattagcc ctatt

<210>  20
<211>  25
<212>  DNA
<213>  artificial sequence

<220>
<223>  POL Second PCR 1st amplicon_Primer 3'

<400>  20
ctttccatcc ctgtggaagc acatt

<210>  21
<211>  29
<212>  DNA
<213>  artificial sequence

<220>
<223>  POL Second PCR 2nd amplicon_Primer 5'

<400>  21
gaaaatccat acaatactcc agtatttgc

<210>  22
<211>  27
<212>  DNA
<213>  artificial sequence

<220>
<223>  POL Second PCR 2nd amplicon_Primer 3'

<400>  22
ctatgctgcc ctatttctaa gtcagat

<210>  23
<211>  18
<212>  DNA
<213>  artificial sequence

<220>
<223>  POL Second PCR 3rd amplicon_Primer 5'

<220>
<221>  misc_feature
<222>  (3)..(3)
<223>  r is A or G

<400>  23
ctrgatgtgg gtgatgca

<210>  24
<211>  20
<212>  DNA
<213>  artificial sequence

<220>
<223>  POL Second PCR 3rd amplicon_Primer 3'

<220>
<221>  misc_feature
<222>  (2)..(2)
<223>  n is A or G or C or T

<220>
<221>  misc_feature
<222>  (3)..(3)
<223>  y is T or C

<400>  24
cnytataggc tgtactgtcc




<210>  25
<211>  9
<212>  PRT
<213>  artificial sequence

<220>
<223>  Pic_ Pol 325-355 HXB2

<400>  25

Ala Ile Phe Gln Ser Ser Met Thr Lys
1               5


<210>  26
<211>  9
<212>  PRT
<213>  artificial sequence

<220>
<223>  Pic_ Pol 325-355 Archived

<400>  26

Ala Ile Phe Gln Ala Ser Met Thr Lys
1               5


<210>  27
<211>  8
<212>  PRT
<213>  artificial sequence

<220>
<223>  Rou_ Gag 1 HXB2 and Archived

<400>  27

Glu Ile Tyr Lys Arg Trp Ile Ile
1               5


<210>  28
<211>  9
<212>  PRT
<213>  artificial sequence

<220>

<223>  Rou_ Gag 2 HXB2 and Archived

<400>  28

Gly Gly Lys Lys Lys Tyr Lys Leu Lys
1               5

<210>  29
<211>  9
<212>  PRT
<213>  artificial sequence

<220>
<223>  Laf_Nef 1 HXB2

<400>  29

Phe Pro Val Thr Pro Gln Val Pro Leu
1               5

<210>  30
<211>  9
<212>  PRT
<213>  artificial sequence

<220>
<223>  Laf_Nef 1 Archived

<400>  30

Phe Pro Val Lys Pro Gln Val Pro Val
1               5

<210>  31
<211>  10
<212>  PRT
<213>  artificial sequence

<220>
<223>  Laf_Nef 2 HXB2

<400>  31

Phe Pro Val Thr Pro Gln Val Pro Leu Arg
1               5                   10

<210>  32
<211>  10
<212>  PRT
<213>  artificial sequence

<220>
<223>  Laf_Nef 2 Archived

<400>  32

Phe Pro Val Lys Pro Gln Val Pro Val Arg
1               5                   10

<210>  33
<211>  9
<212>  PRT
<213>  artificial sequence

<220>
<223>  Laf_Nef 3 HXB2

<400>  33

Thr Pro Gln Val Pro Leu Arg Pro Met
1               5


<210>  34
<211>  9
<212>  PRT

<213>  artificial sequence

<220>
<223>  Laf_Nef 3 Archived

<400>  34

Lys Pro Gln Val Pro Val Arg Pro Met
1               5


<210>  35
<211>  9
<212>  PRT
<213>  artificial sequence

<220>
<223>  Laf_Nef 4 HXB2

<400>  35

Arg Pro Met Thr Tyr Lys Ala Ala Val
1               5


<210>  36
<211>  9
<212>  PRT
<213>  artificial sequence

<220>
<223>  Laf_Nef 4 Archived

<400>  36

Arg Pro Met Thr Tyr Lys Ala Ala Phe
1               5


<210>  37
<211>  9
<212>  PRT
<213>  artificial sequence

<220>
<223>  Laf_Nef 5 HXB2

<400>  37

Arg Pro Met Thr Tyr Lys Ala Ala Leu
1               5

<210>  38
<211>  9
<212>  PRT
<213>  artificial sequence

<220>
<223>  Laf_Nef 5 Archived

<400>  38

Arg Pro Met Thr Tyr Lys Ala Ala Phe
1               5

<210>  39
<211>  9
<212>  PRT
<213>  artificial sequence

<220>
<223>  Eli_Gag HXB2

<400>  39

Arg Leu Arg Pro Gly Gly Lys Lys Lys
1               5

<210>  40
<211>  10
<212>  PRT
<213>  artificial sequence

<220>
<223>  Eli_Gag Archived

<400>  40

Arg Leu Arg Arg Pro Gly Gly Lys Lys Gln
1               5                   10

<210>  41
<211>  9
<212>  PRT
<213>  artificial sequence

<220>
<223>  Pic_RT 1 HXB2 and Archived

<400>  41

Glu Lys Glu Gly Lys Ile Ser Lys Ile
1               5

<210>  42
<211>  8
<212>  PRT

<213>  artificial sequence

<220>
<223>  Pic_RT 2 HXB2

<400>  42

Thr Ala Phe Thr Ile Pro Ser Ile
1               5


<210>  43
<211>  8
<212>  PRT
<213>  artificial sequence

<220>
<223>  Pic_RT 2 Archived

<400>  43


Thr Ala Phe Thr Ile Pro Ser Leu
1               5


<210>  44
<211>  9
<212>  PRT
<213>  artificial sequence

<220>
<223>  Sei_ HLA-A *02:01 _Gag p17 HXB2

<400>  44

Ser Leu Tyr Asn Thr Val Ala Thr Leu
1               5


<210>  45
<211>  9
<212>  PRT
<213>  artificial sequence

<220>
<223>  Sei_ HLA-A *02:01 _RNA



<220>
<221>  MISC_FEATURE
<222>  (3)..(3)
<223>  Xaa is Phe or Tyr

<220>
<221>  MISC_FEATURE
<222>  (6)..(6)
<223>  Xaa is Ile or Val

<400>  45

Ser Leu Xaa Asn Thr Xaa Ser Thr Leu
1               5

<210>  46
<211>  9
<212>  PRT
<213>  artificial sequence

<220>
<223>  Sei_ HLA-A *02:01 _Archived

<220>
<221>  MISC_FEATURE
<222>  (3)..(3)
<223>  Xaa is Tyr or Phe

<220>
<221>  MISC_FEATURE
<222>  (6)..(6)
<223>  Xaa is Val or Ile

<220>
<221>  MISC_FEATURE
<222>  (7)..(7)
<223>  Xaa is Ser or Ala

<220>
<221>  MISC_FEATURE
<222>  (8)..(8)
<223>  Xaa is Ile or Val or Ala or Thr

<400>  46

Ser Leu Xaa Asn Thr Xaa Xaa Xaa Leu
1               5

<210>  47
<211>  10
<212>  PRT
<213>  artificial sequence

<220>
<223>  Sei_ HLA-A *40:01 _Gag p17 HXB2

<400>  47

Ile Glu Ile Lys Asp Thr Lys Glu Ala Leu
1               5                   10

<210>  48
<211>  10
<212>  PRT
<213>  artificial sequence

<220>
<223>  Sei_ HLA-A *40:01 _RNA

<220>
<221>  MISC_FEATURE
<222>  (1)..(1)
<223>  Xaa is Ile or Met

<220>
<221>  MISC_FEATURE

<222>  (3)..(3)
<223>  Xaa is Ile or Val

<400>  48

Xaa Asp Xaa Lys Asp Thr Lys Glu Ala Leu
1               5                   10


<210>  49
<211>  10
<212>  PRT
<213>  artificial sequence

<220>
<223>  Sei_ HLA-A *40:01 _Archived

<400>  49

Ile Asp Val Lys Asp Thr Lys Glu Ala Leu
1               5                   10


<210>  50
<211>  10
<212>  PRT
<213>  artificial sequence

<220>
<223>  Eli_RT 1 HXB2 and Archived

<400>  50

Lys Leu Val Asp Phe Arg Glu Leu Asn Lys
1               5                   10


<210>  51
<211>  9
<212>  PRT
<213>  artificial sequence

<220>
<223>  Eli_RT 2 HXB2

<400>  51

Ala Ile Phe Lys Ser Ser Met Thr Lys
1               5


<210>  52
<211>  9
<212>  PRT
<213>  artificial sequence

<220>
<223>  Eli_RT 2 Archived

<400>  52

Ala Ile Phe Gln Cys Ser Met Thr Lys
1               5

<210>  53
<211>  10
<212>  PRT
<213>  artificial sequence

<220>
<223>  Tre_Gag p17 HXB2

<400>  53

Ile Glu Ile Lys Asp Thr Lys Glu Ala Leu
1               5                   10


<210>  54
<211>  10
<212>  PRT
<213>  artificial sequence

<220>
<223>  Tre_Gag p17 Archived (1)

<400>  54

Ile Asp Val Lys Asp Thr Lys Glu Ala Leu
1               5                   10


<210>  55
<211>  10
<212>  PRT
<213>  artificial sequence

<220>
<223>  Tre_Gag p17 Archived (2)

<400>  55

Ile Asp Val Lys Asp Thr Lys Glu Ala Val
1               5                   10


<210>  56
<211>  9
<212>  PRT
<213>  artificial sequence

<220>
<223>  Tri_ HLA-A *02:01_HXB2 epitope

<400>  56

Val Ile Tyr Gln Tyr Met Asp Asp Leu
1               5


<210>  57
<211>  9
<212>  PRT
<213>  artificial sequence

<220>
<223>  Tri_ HLA-A *02:01 _Archived epitope

<400>  57

Val Ile Tyr Gln Tyr Ile Asp Asp Leu
1               5

## Description

The present invention relates to the field of medicine, and more particularly to the field of the vaccination of HIV infections. It notably relates a method for determining and selecting epitopes susceptible to elicite a T-cell response to HIV.

### BACKGROUND OF THE INVENTION

The human immunodeficiency virus, or HIV, is a retrovirus of the lentivirus genus which infects humans and is responsible for the acquired immunodeficiency syndrome (AIDS). The global spread of HIV and dramatically large number of people infected with the virus have made AIDS a global heath priority.

HIV infection is a chronic infection with non-stop viral replication leading to decrease of the number of TCD₄ lymphocytes and immunodepression. Viral replication can be reduced by antiretroviral drugs of different classes. The reduction of viral replication, generally below the threshold of the viral load commercial assays, is followed by an increase of the TCD₄ lymphocytes. On one side, different clinical trials have shown, in patients at full success, that interruption of an antiretroviral therapy (ART) is followed by a rebound of viral replication. On other side, lifelong adherence to ART medication in HIV-infected patients is not a convincing approach because of important limitations (such as resistance to medication, adverse effects in the medium-to-long-term, cost...).

New therapeutic alternatives to ART therefore still merit investigation. Antiretroviral treatments are beneficial for delaying the appearance of AIDS, but in patients at success of an antiretroviral therapy, the next step is viral eradication.

Classical approaches such as whole inactivated virus or recombinant proteins initially were potentially interesting as therapeutic vaccines. Overall, however, the ability of these early vaccines to increase HIV-specific responses was very limited and study results were discouraging, as no consistent immunogenicity was demonstrated and there was no clear impact on viral load. The understanding of the biology of HIV infection and the role of immune responses in containing viral replication in long-term non-progressor patients needs still to be developed. Capacity of HIV, type 1 in particular, to mutate and evolve rapidly has made combating this virus a tremendous challenge. CD₈ cytotoxic T-lymphocyte (CTL) responses are associated with variable levels of HIV infection control, yet these responses are unable to clear the virus. Viruses are able to evade CTL responses mainly via mutations within the epitopes and selection for escape mutants is a major driving force of HIV evolution. Once viral escape occurs, decay of corresponding CTL responses and generation of responses directed towards mutants and epitopes at new locations have been observed. Examination of the dynamics of primary HIV-1-specific CTL responses in acutely infected subjects has suggested that responses rapidly selected escape mutations and were followed by responses to epitopes that escaped more slowly or were invariant. However, the dynamics of later CTL responses and CTL responses to epitope variants were not clear. Moreover, there is some discordance on whether or not there is a viral evolution under an efficient antiretroviral treatment.

During the course of the AIDS disease, there is normally an initial period of clinical latency which can extend for several years after infection with HIV-1. During this period, there is a chronic replication of HIV-1 in producing cells while the virus exists as a dormant or non-expressing provirus in a reservoir sub-population of chronically infected cells. In Vitro studies with chronically HIV-1-infected tumor cell lines have shown that activation of the dormant provirus occurs when the correct stimuli are encountered. Theoretically, a similar activation occurs with chronically infected cells *in vivo* and results in TCD₄ lymphocytes down-modulation, production of viral progeny, and cytopathic sequela. The TCD₄ lymphocyte is the *in vivo* reservoir for HIV-1.

While the obstacles to creating an effective HIV vaccine are considerable, advances have been made in the last few years with the development of novel vaccines strategies, such as polyepitope vaccines, conserved-region vaccines, central protein design, polyvalent natural proteins or polyvalent central proteins strategies. Most of the HIV vaccine prototypes developed to date have used naturally occurring HIV proteins from circulating virus as vaccine antigens. Preferably, these antigens, either unmodified or with engineered deletions introduced to improve safety or enhance immunogenicity, are close to consensus sequences so as to provide the best coverage of potential epitopes in a target population. Up today, there is no consensus epitope efficient to obtain an optimal vaccination therapy for a population of patients infected with HIV. Accordingly, there is a strong need to methods to determine efficient epitopes susceptible to elicit a T-cell response.

A complementary important aspect to bear in mind when developing a vaccine strategy is the presentation of epitope to immune system, thereby inducing an immune response. Presentation is indeed dependent of binding capacity of an epitope to major histocompatibility complex (MHC) molecules. MHC molecules are classified as either Class I or Class II molecules. Class II MHC molecules are expressed primarily on cells involved in initiating and sustaining immune responses, such as T lymphocytes, B lymphocytes, macrophages, etc. Class II MHC molecules are recognized by helper T lymphocytes and induce proliferation of helper T lymphocytes and amplification of the immune response to the particular immunogenic peptide that is displayed. Class I MHC molecules are expressed on almost all nucleated cells and are recognized by CTLs, which then destroy the antigen-bearing cells. The CTL recognizes the antigen in the form of a peptide fragment bound to the MHC class I molecules rather than the intact foreign antigen itself. The antigen must normally be endogenously synthesized by the cell, and a portion of the protein antigen is degraded into small peptide fragments in the cytoplasm. Some of these small peptides translocate into a pre-Golgi compartment and interact with class I heavy chains to facilitate proper folding and association with the subunit beta 2 microglobulin. The peptide-MHC class I complex is then routed to the cell surface for expression and potential recognition by specific CTLs. The MHC class I antigens are encoded by the HLA-A, B, and C loci. HLA-A and HLA-B antigens are expressed at the cell surface at approximately equal densities, whereas the expression of HLA-C is significantly lower. Each of these loci has a number of alleles. Several authors have provided preliminary evidence that class I binding motifs can be applied to the identification of potential immunogenic peptides in animal models. Because human population groups, including racial and ethnic groups, have distinct patterns of distribution of HLA alleles, it will be of value to identify suitable epitopes capable of binding to HLA allele, so as to achieve sufficient coverage of all population groups. The present invention addresses these needs.

### SUMMARY OF THE INVENTION

The inventors have unexpectedly and surprisingly discovered that the problems and limitations noted above can be overcome by practicing the invention disclosed herein. In particular, the present invention stems on the discovery of an evolution of the virus, at the level of nucleotide sequence, between the plasma of a human subject affected with HIV infection and the proviral intracellular compartments of the same subject. Considering this, the inventors have discovered that it is possible to provide inventive methods to select and design vaccine compositions that address the problems and limitations in the art. It becomes clear that curative vaccination with generic epitopes from circulating virus, as stated in the art, cannot be fully efficient because the epitopes are different or modified when they are archived. Even a reference viral RNA before initiation of ART can be a decoy, and the archived epitopes may be different. The inventors propose a very new approach to identify and select HIV epitopes in vaccine strategy based on selection of HIV provirus epitopes (also called archived epitopes). Assuming that the archived proviral DNA will be the major origin of viral replication at failure or treatment interruption, the vaccine epitopes will be selected from the sequenced proviral DNA in agreement with the HLA alleles, in particular class I alleles, of the patients. Archived provirus epitope suitable to presentation, i.e. corresponding to HLA class I alleles the subject, is then selected to design suitable vaccine based on the provirus epitopes.

The present invention provides a new approach to designing immunogenic vaccinal strategy most effective to elicit a T-cell response in a subject affected with HIV infection. Ultimately, this approach could be combined with the best other strategies to deal with the magnitude, character, and persistence of a vaccine-elicited immune response in order to create an effective HIV vaccine.

In a first aspect, the present invention relates to a method for selecting an epitope susceptible to elicite a T-cell response to human immunodeficiency virus (HIV) in a subject affected with HIV infection, wherein the method comprises:
i) sequencing HIV proviral genome archived in cells of said subject, or part thereof;
ii) identifying one or more proviral epitope(s) and determining binding capacity of said proviral epitope(s) to HLA class I molecules of said subj ect;
iii) selecting proviral epitope(s) presenting a binding capacity to HLA class I molecules, thereby selecting proviral epitope(s)susceptible to elicite a T-cell response to HIV,
the method further optionally comprising a step of molecular typing HLA class I alleles of said subject before step ii).

In still another embodiment, the invention comprises a method for preparing a vaccine composition susceptible to elicit a T-cell response to HIV in a subject, wherein the method comprises the steps i), ii) and iii) as defined above and further comprises preparing a vaccine composition comprising at least one proviral epitope selected in step iii).

In a second aspect, the present invention relates to a method for selecting a subject affected with HIV infection or determining whether a subject affected with HIV infection is susceptible to benefit from a vaccine composition containing one or more epitope(s), wherein the method comprises:
i) sequencing HIV proviral genome archived in cells of said subject,or part thereof, to identify the proviral sequence corresponding to said one or more epitope(s) of said vaccine composition;
ii) comparing proviral sequence(s) of said epitope(s) identified in step i) to epitope(s) of the vaccine composition;
iii) selecting the subject when no significant difference is determined in step ii).

Preferably, the method further comprises:
iv) determining binding capacity of epitope(s) of the vaccine composition to HLA class I molecules of said subject; and
v) selecting the subject when epitope(s) of the vaccine composition present(s) binding capacity to HLA class I molecules of said subject,
the method further optionally comprising a step of molecular typing HLA class I alleles of said subject before step iv).

Preferably, HIV proviral genome sequenced in the methods of the invention is sequenced for the whole proviral genome. Alternatively, HIV proviral genome is sequenced for a part thereof, preferably a region selected from the group consisting of Gag, Nef, Pol, Env regions and combinations thereof.

In a preferred embodiment, HIV proviral genome is a HIV-1 proviral genome.

Preferably, the epitope(s) is/are present in Gag, Nef, Pol and/or Env region(s).

In a preferred embodiment of the invention, the subject affected with HIV infection is at success of antiretroviral therapy.

Preferably, the cells in which HIV proviral genome is archived are selected from circulating cells and viral reservoir tissue and/or cell, more preferably from lymphoid tissue, in particular gut lymphoid tissue, or memory CD4⁺ T cells.

Preferably, the molecular typing of HLA class I alleles is a molecular typing of HLA-A and HLA-B class I alleles.

Optionally, the binding capacity of epitope(s) to HLA class I molecules is a theoretical binding capacity. For instance, the binding capacity of epitope(s) to HLA class I molecules determined in the methods of the invention is realized by means of database, preferably a public database, more preferably the "immune epitope database and analysis resource" (IEDB).

Optionally, the proviral HIV genome sequencing is performed by Sanger sequencing method or by next generation sequencing (NGS) methods, preferably by ultra deep pyrosequencing (UDPS) sequencing method.

### BRIEF DESCRIPTION OF THE DRAWINGS

**Figure 1****:** Patients, antiretroviral drugs and length of treatment, viral subtype, drug resistance mutations, HLA I alleles
**Figure 2****:** Potential CTL reactivity against archived viral epitope according to HLA I alleles: cross reactivity to Lipo5 lipopeptides
**Figure 3****:** Theoretical presentation of CTL epitopes in the sequenced parts of the archived proviral DNA

### DETAILED DESCRIPTION OF THE INVENTION

Importance of T CD8⁺ cytotoxic response for the decrease of viral replication in the primary infection phase of HIV infection is well known. It is moreover now clear that these cellular responses and the corresponding vaccine attempts are dependent on the immunogenetical characters of the individuals, mainly the HLA class I alleles.

The present invention results from the particular interest of the inventors to HIV-1 patients at success of a first line ART (AntiRetroviral Therapy). Generally, these patients are not routinely extensively investigated because they have an undetectable viral load. However, the inventors decided to focus the experiment on proviral DNA in order to address the following question: Taking into account the HLA class I alleles of patients, can the archived viral CTL epitopes be presented to their immunological system, assuming that replication and release from the archived virus will be a major part of the emerging viral replication at failure or interruption of ART ?

By asking this question, the inventors demonstrated that curative vaccination with generic CTL epitopes cannot be fully efficient as such, because proviral epitopes in a patient can be distinct from reference epitopes and evenmore, because proviral epitopes can be modified when archived in genome of a cell. Therefore, the inventors surprisingly found that the sequences of the circulating virus can be distinct from the archived provirus.

Therefore, inventors disclose a method to design vaccine epitopes that will be in agreement with the HLA class I alleles of the patients and with the epitopes present in the provirus. Therefore, selected vaccine epitopes are more susceptible to elicit a T-cell response to the subject affected with HIV infection.

More particularly, a first aspect of the present invention concerns a method for selecting an epitope susceptible to elicit a T-cell response to HIV in a subject affected with HIV infection, wherein the method comprises:
i) sequencing HIV proviral genome archived in cells of said subject, or part thereof;
ii) identifying one or more proviral epitope(s) and determining binding capacity of said proviral epitope(s) to HLA class I molecules of said subj ect;
iii) selecting proviral epitope(s) presenting a binding capacity to HLA class I molecules, thereby selecting proviral epitope(s)susceptible to elicit a T-cell response to HIV,
the method further optionally comprising a step of molecular typing HLA class I alleles of said subject before step ii).

HIV refers to Human Immunodeficiency Virus. In particular, it includes HIV-1 and HIV-2. Preferably, HIV is HIV-1.

By the term "proviral" or "archived", it is intended the sequence(s) or genome of the HIV virus which has been integrated into the chromosomes of patient's cells.

By "subject" or "patient" is intended a human, in particular a human infected by HIV. The subject can be an adult or a child. In a very particular embodiment, the subject or patient is at success of antiretroviral therapy. This means that the subject or patient infected by HIV, after a treatment with an antiretroviral therapy, has a low viral load, preferably below 50 copies/mL.

In one embodiment, the method comprises the sequencing of the whole HIV proviral genome.

Alternatively, only part(s) of HIV proviral genome can be sequenced. Indeed, epitopes generally used for preparing vaccine are present in Gag, Nef, Pol, Vif, Vpr, Vpu and/or Env region(s). Preferably, HIV proviral genome is sequenced for a region selected from the group consisting of Gag, Nef, Pol, Vif, Vpr, Vpu and/or Env region(s) and parts or combination thereof, preferably a region selected from the group consisting of Gag, Nef, Pol, Env regions and parts or combinations thereof, more preferably for the four regions. Still more preferably, HIV proviral genome is sequenced for a region selected from the group consisting of Gag, Nef, Pol and Env regions and parts or combinations thereof, still more preferably for the three regions. By "region" is intended herein which encodes the gene selected from the group consisting of Gag, Nef, Pol, Env genes. The region may comprise the coding sequence and also the regulatory elements of the gene. The preferred region is the coding sequence or part thereof.

As it is well-known, Gag gene encodes the nucleocapsid-core proteins; Pol gene encodes Reverse Transcriptase, protease, integrase and ribonuclease; Env gene encodes viral-coat proteins; Nef gene encodes a Negative Regulatory Factor; Vif gene encodes viral infectivity factor; Vpr gene encodes viral protein R; Vpu gene encodes viral protein unique. In respect to Pol, a part of particular interest is the sequence encoding the Reverse Transcriptase (RT). Alternatively, the portion encoding the protease in Pol may be of particular interest.

The method may comprise an additional preliminary step of providing a sample from the patient. The sample includes cells with archived HIV genome, for instance circulating cells or viral reservoir tissues or cells. In a first embodiment, the sample may be a blood sample, in particular a PBMC (peripheral blood mononuclear cells) sample from the patient. In another embodiment, the sample includes viral reservoir tissues or cells, preferably from lymphoid tissue, in particular gut lymphoid tissue. Preferably, the sample includes memory CD4+ T cells from the patient.

The sequencing step may comprise DNA extraction, PCR amplification and sequencing. The methods of sequencing HIV proviral sequences are well-known to the one skilled in the art.

The sequencing can be performed by any method known in the art, for instance by the well-known Sanger method. However, more effective sequencing methods are now available and known under the designation of next generation sequencing. In a preferred embodiment, the sequencing is performed by using the UDPS method (Ultra-Deep Pyro Sequencing).

Once the proviral sequences have been obtained, the sequences are analyzed in order to identify one or more epitopes and determining their binding capacity to HLA class I molecules of said subject.

In particular, the epitopes under consideration in the present invention are those susceptible to induce a CTL (cytotoxic T lymphocyte) response, i.e., T Cell epitopes binding to HLA class I molecules. These epitopes are herein called CTL epitopes or T cell epitopes. These epitopes are generally peptides of 8-15 amino acids, preferably 8-11 amino acids. Typically, a T cell epitope is 9 amino acids in length.

"Human Leukocyte Antigen" or "HLA" is a human class I or class II Major Histocompatibility Complex (MHC) protein (see, *e.g.,* Stites, et al., IMMUNOLOGY, 8TH ED., Lange Publishing, Los Altos, CA (1994). Class I HLA molecules are expressed on almost all nucleated cells and are recognized by cytotoxic T lymphocytes (CTLs), which then destroy the antigen-bearing cells. CTLs are particularly important in fighting viral infections. The CTL recognizes the antigen in the form of a peptide fragment bound to the HLA class I molecules rather than the intact foreign antigen itself. Antigen presenting systems are preferably allele-phenotyped, in particular HLA-phenotyped. HLA class I molecules include three major groups, namely HLA-A, HLA-B and HLA-C molecules. HLA class I molecules are grouped into such HLA supertypes that share somewhat similar binding affinity for peptides bearing certain amino acid motifs. A non-exhaustive list of HLA supertypes includes: A01; A02; A03; A24; B07; B27; B44; B58 and B62.

In the context of the present invention, the HLA class I alleles to take into consideration are HLA-A, HLA-B and HLA-C alleles, and more preferably HLA-A and HLA-B alleles.

Therefore, if the HLA class I alleles of the patient are unknown, it is necessary to perform the molecular typing of HLA class I alleles of the patient. The methods for molecular typing of HLA class I alleles are well-known by the one skilled in the art.

Then, on the basis of proviral sequences of the subject and its HLA class I alleles, one or more epitopes can be identified or defined in the proviral sequences of the subject and its binding capacity to the HLA class I molecules of said subject can be determined.

In order to identify one or more epitopes in the proviral sequences of the subject, several options are available to the one skilled in the art.

Firstly, numerous epitopes have already been defined and characterized in the art (EP346022; WO97/34621; WO96/20006; EP 888120; WO99/58658; WO0029008; EP1225907; WO0147955; WO0155177; WO0156355; WO02069691; WO03029285; WO04092201; WO06091798; WO07137591). As an illustration, see the review of Llano et al, 2009, HIV Molecular Immunology, section I-A, 3-24, which provides a list of HIV CTL epitopes (Los Alamos Database available on www.hiv.lanl.gov/content/immunology/index.html). Therefore, based on the list of already identified epitopes, the proviral sequences of the subject can be compared in order to identify the sequences of the provirus from the subject corresponding to the already identified epitopes.

The one skilled in the art can choose any of the available tools for identifying one or several proviral epitopes in the obtained proviral sequences of the subject. For instance, such tools are available on IEDB (www.immuneepitope.org), on SYFPEITHI (www.syfpeithi.de/home.htm), and Los Alamos Database (www.hiv.lanl.gov/content/immunology/index.html). These databases allow the identification of CTL epitopes based on a HIV sequence. Optionally, the identification of proviral epitopes takes into consideration the HLA class I alleles of the subject.

Then, the binding capacity of the identified epitopes to the HLA class I alleles of the subject is determined and the epitope(s) having a binding capacity is/are selected. By "having a binding capacity" is intended that the IC50 is less than 500 nM. The IC50 corresponds to Kd. IC50 is the concentration of peptide in a binding assay at which 50% inhibition of binding of a reference peptide is observed. As used herein, "high binding capacity " with respect to HLA class I molecules is defined as binding with an IC₅₀ value of 50 nM or less, and "intermediate binding capacity " is binding with an IC₅₀ value of between 50 and about 500 nM. In a preferred embodiment, the selected epitopes present a high binding capacity.

In one embodiment, the method includes the determination of a theoretical binding capacity of the epitope to the HLA class I alleles of the subject. Several well-known and recognized databases allow the calculation of a binding capacity of epitope sequences to the HLA class I alleles. Non-exhaustively, the following databases can be cited as referenced tools: IEDB (www.immuneepitope.org), on SYFPEITHI (www.syfpeithi.de/home.htm), Los Alamos Database (www.hiv.lanl.gov/content/immunology/index.html); atom.research.microsoft.com/bio/epipred.aspx.

In an alternative or additional embodiment, the binding capacity is determined by an *in vitro* assay. Peptides comprising epitopes are synthesized and tested for their ability to bind to relevant HLA class I proteins. Assays may involve evaluation of peptide binding to purified HLA class I molecules in relation to the binding of a radioiodinated reference peptide. Alternatively, cells expressing empty class I molecules (*i.e*. cell surface HLA molecules that lack any bound peptide) may be evaluated for peptide binding by immunofluorescent staining and flow microfluorimetry.

Optionally, when variability is observed among the proviral epitope sequences of the subject, the less variable epitopes are selected. In other word, the epitopes which are the most conserved in the proviral sequences of the subject will be selected.

In addition to the determination of the binding capacity of the epitopes, the method may further comprise a step in which the CTL response is measured.

The CTL response can be tested *in vitro* by any CTL assay available in the art. Conventional assays utilized to detect T cell responses include proliferation assays, lymphokine secretion assays, direct cytotoxicity assays, and limiting dilution assays. For instance, the assay may be selected among the well-know IFN-γ ELISpot assay, the CTL lysis assay using standard ⁵¹Chromium release assay, the Live Virus ELISPOT (LVE) assay or the Viral suppression assay (VSA) (see for instance, Ranasinghe et al, 2011, PLoS Pathogens, 7, e1001341).

Also disclosed is a method for preparing a vaccine composition susceptible to elicit a T-cell response to HIV in a subject, wherein the method comprises the selection of proviral epitope(s) susceptible to elicit a T-cell response to HIV in a subject affected with HIV infection as defined above and further comprises preparing a vaccine composition comprising at least one selected proviral epitope. The prepared vaccine composition may include any pharmaceutically acceptable carrier or excipient. Preferably, it comprises several selected epitopes, in particular from 2 to 30 proviral epitopes. In a preferred embodiment, epitopes will be selected in order to be specific of several distinct targets of HIV (e.g., preferably Gag, Nef, Pol, Env regions). Optionally, several epitopes can be selected for each HIV target. Of course, the vaccine composition may further comprise additional components such as vaccine adjuvant (*e.g*., incomplete Freund's adjuvant), liposomes, HTL (helper T lymphocyte) epitopes, antigen-presenting cells, etc. The epitope peptides can be modified to become lipopeptides.

In a particular embodiment of the methods detailed above, the method may further include a step (by Sanger and/or NGS sequencing) of determining if mutation(s) which have been disclosed as Drug Resitstance Mutations is/are present in the proviral sequences of the subject. Such mutations are well-known in the art. For instance, some are disclosed in WO06091798.

In another aspect, the present invention concerns a method for selecting a subject affected with HIV infection or determining whether a subject affected with HIV infection is susceptible to benefit from a vaccine composition containing one or more epitope(s), wherein the method comprises:
i) sequencing HIV proviral genome archived in cells of said subject,or part thereof, to identify the proviral sequence corresponding to said one or more epitope(s) of said vaccine composition;
ii) comparing proviral sequence(s) of said epitope(s) identified in step i) to epitope(s) of the vaccine composition;
iii) selecting the subject when no significant difference is determined in step ii).

By "no significant difference" is intended that the differences have no impact on the binding capacity to HLA class I molecules and/or the capacity of cross-reacting in the CTL response. The binding capacity can be determined by either a theoretical IC50 or an *in vitro* binding assay. The cross-reaction can be easily tested with a CTL response assay. More particularly, it is intended that the proviral sequence when compared to the vaccine epitope presents no more than 3, 2 or 1 amino acid substitutions, preferably less than 2 or 1 amino acid substitutions.

Optionally, the method further comprises:
iv) determining binding capacity of epitope(s) of the vaccine composition to HLA class I molecules of said subject; and
v) selecting the subject when epitope(s) of the vaccine composition present(s) binding capacity to HLA class I molecules of said subject,
the method further optionally comprising a step of molecular typing HLA class I alleles of said subject before step iv).

The method can further comprise a step of determining whether proviral sequence(s) of said epitope(s) present(s) a binding capacity to HLA class I molecules of said subject, and selecting the subject when proviral sequence(s) of said epitope(s) present(s) binding capacity to HLA class I molecules of said subject.

One can thereby, relative to a pre-established vaccine composition, select a subject affected with HIV infection for being a good responder to the vaccine therapy, or determining whether the subject affected with HIV infection is susceptible to benefit from the vaccine therapy.

### EXAMPLES

### MATERIALS AND METHODS

### Study patients

Eleven HIV-1 infected patients were enrolled in this study which received a positive agreement from the « Comité de protection des personnes du Sud Ouest » (DC 2012/48). All are adults were at success of a first ART including at least one NRTI and/or NNRTI (Non-Nucleoside Reverse Transcriptase Inhibitors and Non-Nucleoside Reverse Transcriptase Inhibitors). Written informed consent was obtained from each study participant. First line ART period ranges from 8 months to 9 years with indetectable viral load (under 50 copies/ml Roche Ampliprep Cobas Taqman and under 40 copies/ml Abbott) and without intermittent viremia or blip.

### DNA extraction and PCR amplification.

PBMCs were separated by density gradient centrifugation and stored as dry pellets at -80°C. PBMC HIV-1 DNA was extracted using the MagNA Pure automatic system (Roche) according to the manufacturer's specifications, and quantitated by UV optical density measurement. PBMC DNA extract was used for HIV-1 proviral and episomic DNA quantification by means of a standardized real-time PCR technique, based on the 5' nuclease method, in the long terminal repeat region. This technique takes both integrated HIV-1 provirus and non-integrated HIV-1 DNA forms into account. Results were expressed as the log10-transformed number of HIV-1 DNA copies/106 PBMCs, with a detection threshold of 1.8 log10 copies/106 PBMCs.

Epitopic regions of Gag and Nef were amplified from DNA extracted previously using primers described in Table 1 and AmpliTaq Gold with GeneAmp Kit (Applied Biosystem, Foster City, CA). Epitopic region of RT was amplified using primers described in Table 1 and FastStart Taq DNApol HiFi (Roche).

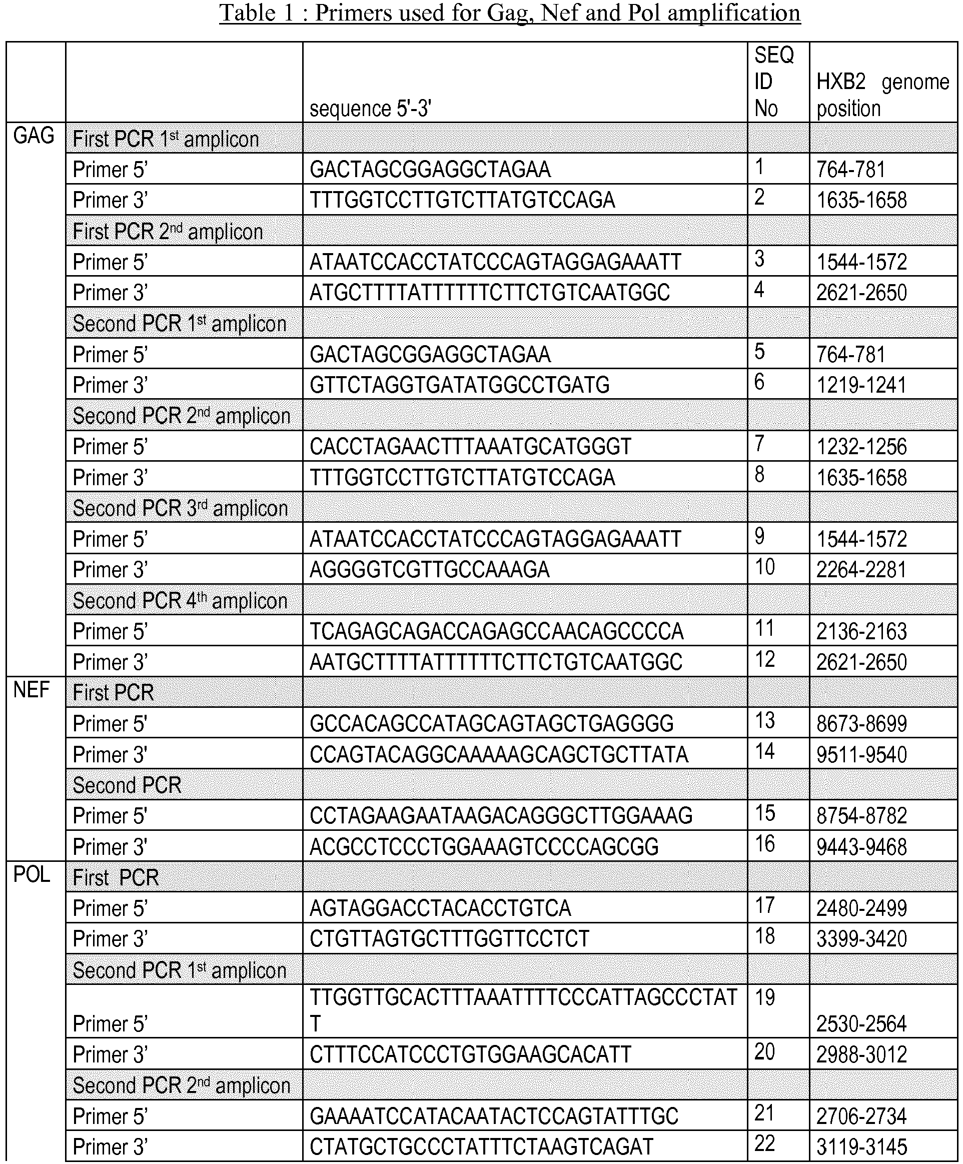

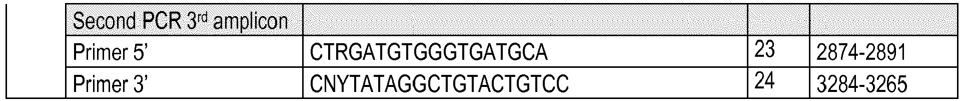

### HLA class I typing

Genomic DNA was extracted from the frozen white blood cell pellets and quantitated as described above. Intermediate-to-high resolution was performed by reverse Polymerase Chain Reaction-Sequence Specific Oligonucleotide (PCR-SSO) hybridization using the LuminexH flow beads LabTypeH assay (InGen, Chilly-Mazarin, France) for the A and B loci. Allelic ambiguities were solved with PCR-Sequence Specific Primer (SSP) amplification using Olerup assays (BioNoBis, Montfort L'Amaury, France). The manufacturers' recommendations were strictly followed. Allele assignment was performed by comparison with the official nomenclature and validated by the WHO committee for HLA system factors.

### Gag, Nef and Pol Sanger sequencing

PCR products were sequenced on both strands using an Applied Biosystems 3500xls Dx Genetic Analyzer and primers from the second PCR.

### RT Ultra-deep pyrosequencing

RT UDPS was performed using the Roche GS Junior equipment. Amplicons previously obtained, purified and quantified were pooled at equimolar concentrations. Clonal amplification on beads (EmPCR) was performed using the 454 Life Science reagents that enable bidirectional sequencing, composed of 30 cycles PCR amplification. DNA containing beads were recovered and UDPS was performed on the GS Junior sequencer (454 Life Sciences; Roche). UDPS generated a median of 11.000 sequence reads per sample.

### Immune recognition tools

The immune epitope database (www.immuneepitope.org) was used to predict T-cell epitope recognition. We have chosen to evaluate the affinity of the epitopes for the MHC molecules by the MHC IC50 (nM) value; small values are associated with better binders and, particularly, 500 nM is often used as the threshold between binders and non-binders.

### RESULTS

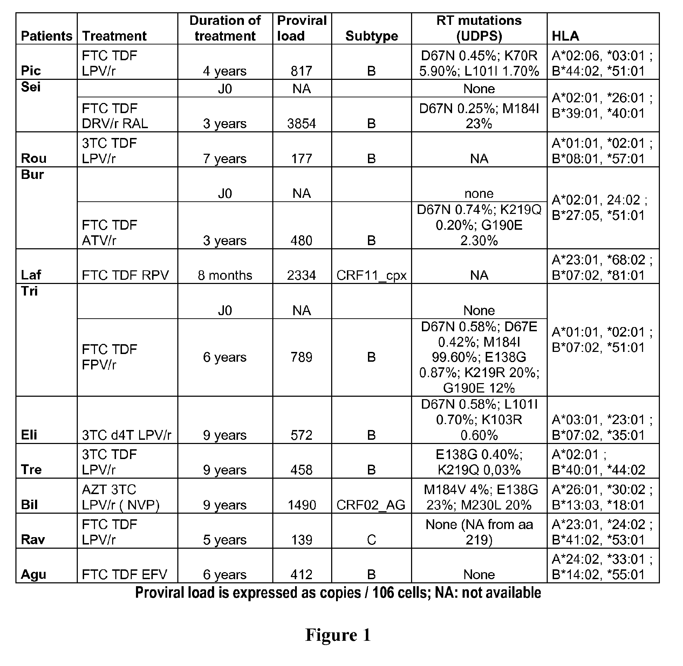

### Patients and antiretroviral treatment (Figure 1)

11 patients have been recruited; all were receiving a successful first line ART 8 months to 9 years after initiation of treatment; no case exhibited a blip all along the survey period reported; all treatments included at least one drug of the NRTI/NNRTI classes.

### Molecular characterization of HIV-1 and viral loads (Figure 1)

Among 11 HIV-1 isolates, all RNA viral loads (VL) were below the threshold of 50 copies/Ml; For all samples, the quantitation of 2-LTR episomic DNA was found below the threshold of 10 copies/million PBMC.

The Gag sequence of one strain (Agu) exhibited 5 stop codons; in all cases, tryptophan amino acid was replaced by a stop codon, consequence of a switch from TGG (wild) to TAG or TAA.

### Drug resistance mutations (DRMs) analyzed by ultradeep pyrosequencing (UDPS) (Figure 1)

When we consider the frequencies of mutant variants above 1%, 3 provirus exhibited M184I/V mutations between 4 and 99.6%; 2 provirus were bearing G190E variants (2.30 and 12% respectively), one 5.90% K70R and one 20% M230L. 2 isolates were bearing two mutations simultaneously: Tri with M184I and G190E and Bil with M184V plus M230L. For three patients (Sei, Bur and Tri), viral RNA could be investigated before initiation of ART, and exhibited DRMs in the proviral DNA, while no DRM could be observed in the initiation sample.

### Potential CTL reactivity against archived viral epitopes according to HLA I alleles

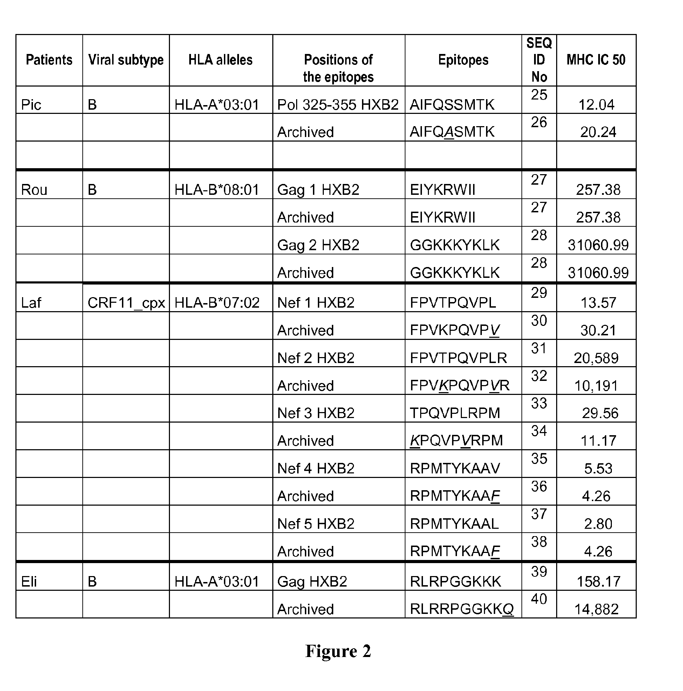

*Cross reactivity to Lipo5 lipopeptides* (Figure 2) The ANRS HIV-LIPO-5 lipopeptides are composed of the Gag 17-35, Gag 253-284, Nef 66-97, Nef 116-145, and Pol 325-355 peptides ( Gahery et al., Thérapie, 2005, 60;243-248). According to the different HLA I alleles and the different available sequences of Gag, Nef and Pol obtained after Sanger sequencing, the data are shown in Figure 2.

**Pic.** The virus has been identified as subtype B HIV-1; with an HLA A*03:01 allele, the patient should recognize the Pol 325-355 epitope (AIFQSSMTK), which is one of the Lipo5 peptides designed from the HXB2 HIV-1 subtype B reference. The archived epitope in the provirus exhibits a substitution (AIFQ*A*SMTK), but the presentation with the allele is still excellent (MHC IC50 20.24 versus 12.04).

**Rou.** The virus is of subtype B; with HLA B*08:01. 2 Gag epitopes can be presented according to the HXB2 reference; EIYKRWII with a MHC IC50 of 257.38 and GGKKKYKLK with a MHC IC50 of 31,060.99 (low affinity). The archived epitopes are identical.

**Laf.** The patient is infected with a CRF11_cpx; with HLA allele B*07:02. 5 epitopes of Nef 66-97 should be recognized. All corresponding archived epitopes are different from those referenced from HXB2 and 4 are efficiently presented while one is not: FPVTPQVPL (MHC IC50 13.57) / FPV*K*PQVP*V*(MHC IC50 30.21); FPVTPQVPLR (MHC IC50 20,589) / FPV*K*PQVP*V*R (MHC IC50 10,191); TPQVPLRPM (MHC IC50 29.56) / *K*PQVP*V*RPM (MHC IC50 11.17); RPMTYKAAV (MHC IC50 5.53) /RPMTYKAA*F* (MHC IC50 4.26) ; RPMTYKAAL (MHC IC50 2.80) / RPMTYKAA*F* (MHC IC50 4.26).

**Eli.** The virus is of subtype B. According to HLA alleles A*03:01, B*07:02 and B* 35:01, 13 epitopes corresponding to Gag 17-35, Gag 253-284, Nef 66-97 and Pol 325-355 could be evaluated (not shown). Most of them exhibit substitutions in the proviral DNA without variation of the MHC IC50. One is no longer recognized: Gag 17-35 ; RLRPGGKKK (MHC IC50 158.17) / RLRRPGGKK*Q* (MHC IC50 14,882).

### Theoretical presentation of CTL epitopes in the sequenced parts of the archived proviral DNA

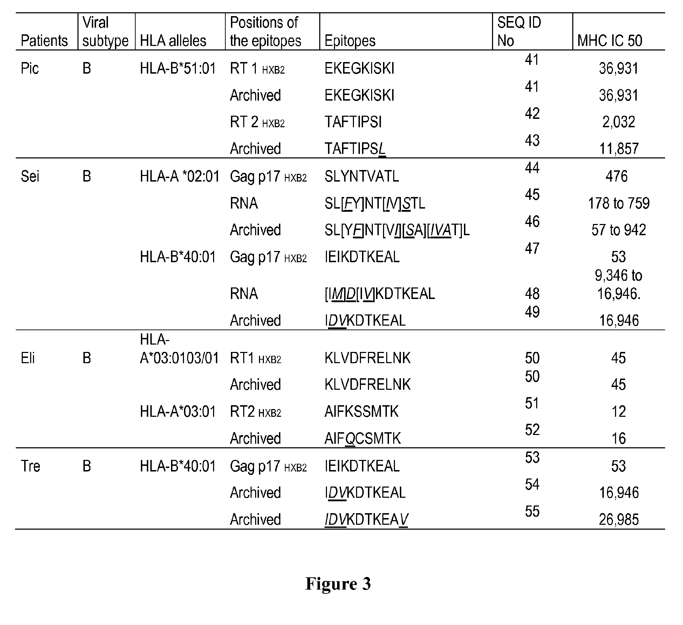

### Different patterns could be noted (Figure 3).

**Pic.** According to HLA allele A*03:01, the epitopes RT (Reverse Transcriptase) 33-43, RT 73-82, RT 93-101 of HXB2 can be presented with MHC IC50 ranging from 45.58 to 367.82. The epitopes which are archived in the proviral DNA are identical. With HLA allele B 51:01, 2 epitopes of HXB2, RT 42-50 (EKEGKISKI) and RT 128-135 (TAFTIPSI), can be presented with MHC IC50 of 36,931 and 2,032 respectively. The first archived epitope is unchanged and the second exhibits a substitution (TAFTIPS*L*) with a MHC IC50 switching from 2,032 to 11,857.

**Sei.** This patient could be investigated on viral RNA before initiation of HAART and on proviral DNA at success. With HLA alleles A*02:01, A*26:01, B*39:01 and B*40:01, 10 epitopes could be analyzed in Gag, Nef and Pol (RT). When HXB2 reference and viral RNA are compared, 6 epitopes are strictly identical, 2 are mutated (with increase of MHC IC50) and 2 exhibit double populations at some codons and corresponding residues.

With HLA allele A*02:01, Gag p17 77-85 (SLYNTVATL) is presented with a MHC IC50 of 476. The viral RNA presents the epitope SL[*F*Y]NT[*I*V]*S*TL with 4 different combinations and MHC IC50 ranging from 178 to 759. In the same way, with HLA allele B 40:01, the epitope Gag p17 92-101 IEIKDTKEAL is recognized with a MHC IC50 of 53; the corresponding epitopes of the viral RNA exhibits 4 combinations [*I*M]*D*[I*V*]KDTKEAL with MHC IC50 ranging from 9,346 to 16,946.

In the proviral DNA at success, the epitopes are identical to those recorded in the viral RNA with 2 exceptions: one is mutated (Nef 92-100 presented by the B*40:01 allele) with a MHC IC50 increasing from 63 to 198; the other is related to Gag p17 92-101 which exhibits 4 different combinations in the viral RNA; the archived epitope is that showing the highest MHC IC50 (I*DV*KDTKEAL; MHC IC50 16,946).

**Eli.** For this subtype B and according to HXB2 reference, 2 epitopes are presented by the HLA allele A*03:01. In the proviral DNA, the first epitope is unchanged and the second is mutated without significant variation of the MHC IC50.

**Tre.** This patient, who is infected with a subtype B HIV-1, is characterized by the HLA alleles A*02:01, B*40:01 and B*44:02. The B*40:01 antigen is able to present the Gag p17 epitope of HXB2 (IEIKDTKEAL) with a MHC IC50 of 53. The archived combinations of this epitope (I*DV*KDTKEAL and I*DV*KDTLEA*V*) exhibit MHC IC50 values of 16,946 and 26,985 respectively.

**Tri.** One epitope matching with HLA allele A*02:01 could be studied by Sanger and UDPS at baseline (RNA) and at success (archived DNA). The HXB2 epitope is VIYQYMDDL (SEQ ID No 56) and the observed epitope in the viral RNA at baseline is identical with a MHC IC50 of 1946.61. At success, the archived epitope is mutated (VIYQY*I*DDL- SEQ ID No 57) with a MHC IC50 of 855.38. The UDPS analysis of the epitope shows that there is a high stability of both epitopes at baseline and at success within the subpecies.

### DISCUSSION

The inventors have analysed the CTL epitopes of the provirus according to the HLA I alleles which determine the presentation of these epitopes to the cytotoxic CD8 lymphocytes. When they consider the lipopeptides which have been designed by ANRS (Lipo5) and if they state that they have to be identical or very close to the archived corresponding epitopes in order to trigger a significant vaccinal therapeutic response, it is clear that, with the partial results they could obtain, there is a high variability of the patterns; some archived epitopes are identical while some are different with a predicted less efficient vaccinal effect. This French therapeutic vaccine has been well tolerated in a phase 2 study (Salmon-Céron et al, AIDS 2010, 24, 2211-2223) and has some interest (Pialoux et al, Clin Vaccine Immunol, 2008, 15, 562-568), but it is highly doubtful that these generic epitopes which have been designed on a HXB2 reference will be identical to archived epitopes.

When the inventors consider the epitopes of the archived provirus and the HLA I alleles, they see that the patterns are also different with epitopes identical to the HXB2 reference and the expected MHC, but also mutated epitopes which are not any longer recognized. Very interesting cases are those where epitopes are recorded as combinations in the viral RNA before initiation of ART, and epitopes finally selected in the proviral DNA are the less efficiently presented. Moreover, the present results on UDPS sequences of a part of RT show that there is an evolution of the virus at the level of nucleotide sequence between the plasma and the proviral intracellular compartments.

Although they have been obtained by simulation and not by biological assays (for example ELIspot) which could hardly be used on a large scale, the present results show that curative vaccination with generic epitopes, mainly CTL epitopes, cannot be fully efficient because the epitopes are different from the B reference or are modified when they are archived as it has been described (Queen et al, J Viro, 2011, 85, 9167-9175) and that the inventors are not in a position of expecting a cross reaction; secondly, these generic epitopes are not systematically suitable to presentation due to the diversity of class I antigens and corresponding HLA alleles. This is a problem not only for LIPO-5 peptides but also for all other generic vaccines based on recombinant viruses or viral DNA (Garcia et al, Hum Vaccin Immunother, 2012, 8, 569-581). On the other hand, when you first identify the HLA I alleles and design potential peptide epitopes on the HXB2 reference, you have to be aware that some of these epitopes can be different in the archived provirus. Even the reference of viral RNA before initiation of HAART can be a decoy because the archived epitopes may be different. The inventors propose that, assuming that the archived proviral DNA will be the major origin of viral replication at failure or treatment interruption, the vaccinal epitopes will be selected from the sequenced proviral DNA in agreement with the HLA alleles of the patients.

### REFERENCES

Data and tools within the IEDB are presented as a public resource: see www.immuneepitope.org and Vita R, Zarebski L, Greenbaum JA, Emami H, Hoof I, Salimi N, Damle R, Sette A, Peters B. The immune epitope database 2.0. Nucleic Acids Res. 2010 Jan;38(Database issue):D854-62. Epub 2009 Nov 11.

## Claims

1. A method for selecting an epitope is susceptible to elicit a T-cell response to human immunodeficiency virus (HIV) in a subject affected with HIV infection, wherein the method comprises:
i) sequencing HIV proviral genome archived in cells of said subject, or part thereof;
ii) identifying one or more proviral epitope(s) and determining binding capacity of said proviral epitope(s) to HLA class I molecules of said subj ect;
iii) selecting proviral epitope(s) presenting a binding capacity to HLA class I molecules, thereby selecting proviral epitope(s) susceptible to elicit a T-cell response to HIV,
the method further optionally comprising a step of molecular typing HLA class I alleles of said subject before step ii).

2. A method for selecting a subject affected with HIV infection or determining whether a subject affected with HIV infection is susceptible to benefit from a vaccine composition containing one or more epitope(s), wherein the method comprises:
i) sequencing HIV proviral genome archived in cells of said subject,or part thereof, to identify the proviral sequence corresponding to said one or more epitope(s) of said vaccine composition;
ii) comparing proviral sequence(s) of said epitope(s) identified in step i) to epitope(s) of the vaccine composition;
iii) selecting the subject when no significant difference is determined in step ii).

3. The method according to claim 2, further comprising :
iv) determining binding capacity of epitope(s) of the vaccine composition to HLA class I molecules of said subject; and
v) selecting the subject when epitope(s) of the vaccine composition present(s) binding capacity to HLA class I molecules of said subject,
the method further optionally comprising a step of molecular typing HLA class I alleles of said subject before step iv).

4. A method for preparing a vaccine composition susceptible to elicite a T-cell response to HIV in a subject, wherein the method comprises the steps i), ii) and iii) as defined in claim 1 and further comprises preparing a vaccine composition comprising at least one proviral epitope selected in step iii).

5. The method according to any of the preceding claims, wherein HIV proviral genome is sequenced for whole proviral genome.

6. The method according to claims 1 to 4, wherein HIV proviral genome is sequenced for a region selected from the group consisting of Gag, Nef, Pol, Env regions and combinations thereof.

7. The method according to any of the preceding claims, wherein HIV proviral genome is a HIV-1 proviral genome.

8. The method according to any of the preceding claims, wherein the epitope(s) is/are present in Gag, Nef, Pol and/or Env region(s).

9. The method according to any of the preceding claims, wherein the subject affected with HIV infection is at success of antiretroviral therapy.

10. The method according to any of the preceding claims, wherein the cells in which HIV proviral genome is archived are selected from circulating cells and viral reservoir tissue and/or cell, more preferably from lymphoid tissue, in particular gut lymphoid tissue, or memory CD4⁺ T cells.

11. The method according to any of the preceding claims, wherein the molecular typing of HLA class I alleles is a molecular typing of HLA-A and HLA-B class I alleles.

12. The method according to any of the preceding claims, wherein the binding capacity is a theoretical binding capacity.

13. The method according to any of the preceding claims, wherein the binding capacity of epitope(s) to HLA class I molecules is determined by means of database, preferably a public database, more preferably the "immune epitope database and analysis resource" (IEDB).

14. The method according to any of the preceding claims, wherein proviral HIV genome sequencing is performed by Sanger sequencing method or next generation sequencing (NGS) method, preferably by ultra deep pyrosequencing (UDPS) sequencing method.

15. The method according to claim 14, wherein proviral HIV genome sequencing is preferably performed by next generation sequencing (NGS) methods, preferably by ultra deep pyrosequencing (UDPS).
